# EUROPÄISCHE PATENTSCHRIFT

(11) **EP 1 727 842 B1**
(45) Veröffentlichungstag und Bekanntmachung des Hinweises auf die Patenterteilung: **17.12.2008**
(21) Anmeldenummer: 05715873.5
(22) Anmeldetag: 09.03.2005
(51) Int. Cl.: C08G 18/02, C08G 18/79

(54) **VERFAHREN ZUR HERSTELLUNG VON ISOCYANURATGRUPPEN AUFWEISENDEN POLYISOCYANATEN UND IHRE VERWENDUNG**
METHOD FOR THE PRODUCTION OF POLYISOCYANATES COMPRISING ISOCYANURATE GROUPS AND USE THEREOF
PROCEDE DE PREPARATION DE POLYISOCYANATES CONTENANT DES GROUPES ISOCYANURATE ET UTILISATION DESDITS COMPOSES

(30) Priorität: 12.03.2004 DE 102004012571
(43) Veröffentlichungstag der Anmeldung: 06.12.2006
(73) Patentinhaber: BASF SE, 67056 Ludwigshafen (DE)
(72) Erfinder: BINDER, Horst, 68623 Lampertheim (DE); GRAF, Hubert, 67487 St. Martin (DE); BRAUN, Frank, 67150 Niederkirchen (DE); SPIES, Bernd, 67063 Ludwigshafen (DE); KIESLICH, Heinrich, 67071 Ludwigshafen (DE)
(86) Internationale Anmeldenummer: PCT/EP2005/002483
(87) Internationale Veröffentlichungsnummer: WO 2005/087828

(56) Entgegenhaltungen:
- EP-A- 0 630 928
- DE-A1- 2 631 733
- US-A- 5 691 440

## Beschreibung

Die vorliegende Erfindung betrifft ein neues Verfahren zur Herstellung von Isocyanuratgruppen aufweisenden Polyisocyanaten durch eine partielle Trimerisierung von (cyclo)-aliphatischen Diisocyanaten in Gegenwart mindestens eines Trimerisierungskatalysators aus der Gruppe tetrasubstituierter Ammonium α-Hydroxycarboxylate und die Verwendung der so erhältlichen Isocyanuratgruppen aufweisenden Polyisocyanate als Polyisocyanatkomponente in Polyurethanlacken.

Verfahren zur partiellen oder vollständigen Trimerisierung von organischen Polyisocyanaten zur Herstellung von Isocyanuratgruppen aufweisenden Polyisocyanaten oder zelligen oder kompakten Isocyanuratgruppen aufweisenden Polyurethanen sind bekannt und werden in zahlreichen Literaturveröffentlichungen beschrieben.

Aus DE-OS 29 16 201 sind Trimerisierungskatalysatoren aus einem quaternären, gegebenenfalls substituierten 2-Hydroxyethylammoniumkation und Säuren als Anion bekannt. In diesen Säuren- R'-COO kann R' einen "gegebenenfalls oxyalkylhaltigen C-C-Alkylrest" bedeuten, für dieses Substitutionsmuster werden jedoch keine Reste R' offenbart. Präzisiert wird dies in dem US-Äquivalent US 4,454,317, in dem der Rest R' beschrieben wird als ein C₁-C₁₂-Alkylrest, der optional mit einer Hydroxyalkylgruppe substituiert ist, d.h. eine Hydroxygruppe befindet sich nähestens in β-Position zur Carboxylgruppe. Einzig Cyanessigsäure und Dichloressigsäure werden als substituierte Säuren in den Beispielen erwähnt.

US 3,862,150 beschreibt Salze aus tertiären Aminen und α-substituierten Carbonsäuren als thermisch zersetzbare Katalysatoren, beispielsweise zur Urethanbildung, in denen als α-Substituenten Nitril-, Sulfonyl-, Sulfuryl-, Carbonyl-, Nitro- Acetyl- und Benzoylgruppen möglich sind. Durch die sich herausbildenden 1,3-Dicarbonylsysteme bzw. carbonylanalogen Systeme findet eine Decarboxylierung vereinfacht statt, so daß derartige Katalysatoren leicht desaktivieren, was den Temperaturbereich für deren Einsetzbarkeit nachteilig einschränkt.

DE-OS 26 31 733 offenbart gegebenenfalls substituierte 2-Hydroxyethyl-alkyl-ammoniumcarbonate oder -carboxylate. Als mögliche Carboxylate sind u.a. Salze der Hydroxyessigsäure (Glykolsäure) offenbart.

Nachteil solcher 2-Hydroxyethyl-alkyl-ammonium carboxylate ist jedoch deren geringe thermische Belastbarkeit, so daß die Anwendung solcher Salze als Katalysatoren auf ein relativ enges Temperaturfenster beschränkt ist und ihre geringe katalytische Wirksamkeit, die zu Verfärbungen des Endprodukts führen kann.

WO 93/12153 beschreibt als Trimerisierungskatalysatoren für Isocyanate wirksame Reaktionsprodukte von Mischungen aus Carbonsäuren, tertiären Aminen und Epoxiden. Beispielhaft wird eine Mischung aus Milchsäure, Bis(3-Dimethylamino)formamid und Phenylglycidylether beschrieben.

Nachteilig an solchen Reaktionsprodukten ist, daß man zu deren Herstellung eine bis zu dreikomponentige Mischung dosieren muß, bei deren optimaler Wirksamkeit es auf eine genaue Einhaltung der Stöchiometrie ankommt, so daß die Gefahr einer Fehldosierung gegeben ist, und daß es sich auch hier wohl um 2-Hydroxyethyl-alkyl-ammonium carboxylate handeln könnte, deren Nachteile bereits oben beschrieben sind.

WO 03/20784 offenbart ein Verfahren zur Herstellung fester, urethanmodifizierter Polyisocyanuratschäume, in dem ein handelsüblicher Trimerisierungskatalysator, beispielsweise Alkalimetallsalze von organischen Säuren, gegebenenfalls in Gegenwart weiterer Trimerisierungskatalysatoren, beispielsweise tertiärer Amine, in Gegenwart einer Carbonsäure und in Gegenwart eines Blähmittels eingesetzt werden. Als Carbonsäuren werden beispielsweise Zitronensäure, Bishydroxypropionsäure, Äpfelsäure und bevorzugt Milchsäure offenbart.

Die Carbonsäure hat keine katalytische Wirkung im Trimerisierungsprozeß, sondern ist lediglich ein Additiv für den Schäumungsprozeß.

J. Robins und D.R. O'Keefe beschreiben in Advances in Urethane Science and Technology, 1981, 8, 185 - 216 Alkalimetalllactat, besonders Kaliumlactat, als verkapselten Katalysator zur Trimerisierung. Nachteilig ist jedoch die Anwesenheit von Wasser, bedingt durch die Herstellung aus der freien Säure und einem Alkalimetallhydroxid, im Isocyanat, da es mit den NCO-Gruppen zu Carbaminsäuregruppen reagieren kann, die nach Decarboxylierung Amine bilden, die wiederum zur Bildung von unerwünschten, weil meist unlöslichen, Harnstoffen führt, und die Allophanatbildung, bedingt durch das verwendete Lösungsmittel Glycerin. Zudem desaktivieren die Katalysatoren schnell (Fig. 7, ebda.)

JP-A 2002-97244 beschreibt die Bildung von Isocyanuraten aus Isocyanaten mit einem gängigen Trimerisierungskatalysator in Gegenwart von Ascorbinsäure.

Hier ist jedoch wiederum die Zugabe von 2 Komponenten, nämlich Katalysator und CoKatalysator, erforderlich, so daß Fehldosierungen möglich sind. Zudem führt JP-A 2002-97244 in Absatz [0020] aus, daß Ascorbinsäure unlöslich in dem Reaktionsmedium ist und dispergiert werden muß, wohingegen der Isocyanurat-Katalysator löslich ist, was die Dosierung zum Erzielen einer optimalen Wirkung nochmals erschwert.

Die Aufgabe der vorliegenden Erfindung bestand darin, einen Katalysator zur Herstellung von im wesentlichen farblose Isocyanuratgruppen enthaltende Polyisocyanaten nach einem möglichst einfachen Verfahren in sehr guter Qualität und unabhängig von deren Herstellung reproduzierbar bereitzustellen, der über einen breiten Temperaturbereich anwendbar ist und der eine einheitliche Struktur aufweist.

Diese Aufgabe wurde gelöst durch ein Verfahren zur Herstellung von Isocyanuratgruppen-enthaltenden Polyisocyanaten durch zumindest teilweise Trimerisierung von (cyclo)aliphatischen Diisocyanaten in dem man die Umsetzung in Gegenwart mindestens eines Trimerisierungskatalysators ausgewählt aus der Gruppe der mit vier Kohlenwasserstoffresten substituierten Ammoniumsalze von α-Hydroxycarboxylaten durchführt.

Kohlenwasserstoffreste sind dabei Substituenten, die ausschließlich aus Kohlenstoff- und Wasserstoffatomen bestehen.

Ein bevorzugter Gegenstand der Erfindung ist ein Verfahren zur Herstellung von Isocyanuratgruppen aufweisenden Polyisocyanaten durch zumindest partielle Trimerisierung von aliphatischen oder/und cycloaliphatischen Diisocyanaten in Gegenwart mindestens eines Trimerisierungskatalysators und anschließend gegebenenfalls Desaktivierung des Trimerisierungskatalysators nach Erreichen des angestrebten Trimerisierungsgrads, in dem man als Trimerisierungskatalysator mindestens ein tetrasubstituiertes Ammonium α-Hydroxycarboxylat der Formel (1) einsetzt, 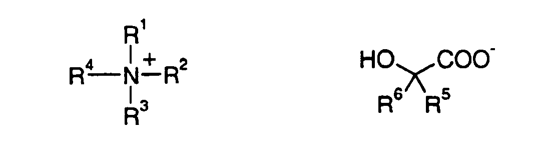 worin
R¹, R², R³, R⁴, R⁵ und R⁶ unabhängig voneinander jeweils gleich oder verschieden sein können und für eine geradkettige oder verzweigte C₁- bis C₂₀-Alkylgruppe, eine gegebenenfalls substituierte C₅- bis C₁₂-Cycloalkylgruppe, eine gegebenenfalls substituierte C₇- bis C₁₀-Aralkylgruppe, oder eine gegebenenfalls substituierte C₆-C₁₂-Arylgruppe stehen oder zwei oder mehr der Reste R¹ bis R⁴ gemeinsam miteinander eine 4-, 5- oder 6-gliedrige Alkylenkette bilden oder zusammen mit einem Stickstoffatom einen 5-oder 6-gliedrigen Ring bilden, der noch ein zusätzliches Stickstoff- oder Sauerstoffatom als Brückenglied enthalten kann oder gemeinsam ein mehrgliedriges, vorzugsweise sechsgliedriges Mehrringsystem, vorzugsweise Zweiringsystem bilden, das noch ein oder mehrere zusätzliche Stickstoffatome, Sauerstoffatome oder Stickstoff- und Sauerstoffatome als Brückenglieder enthalten kann und
R⁵ und R⁶ zusätzlich Wasserstoff, gegebenenfalls durch ein oder mehrere Sauerstoff- und/oder Schwefelatome und/oder ein oder mehrere substituierte oder unsubstituierte Iminogruppen unterbrochene oder durch funktionelle Gruppen, Aryl, Alkyl, Aryloxy, Alkyloxy, Halogen, Heteroatome und/oder Heterocyclen substituierte C₁-C₂₀-Alkyl oder C₆ bis C₁₂-Aryl
sein können.

Darin bedeuten
eine geradkettige oder verzweigte C₁- bis C₂₀-Alkylgruppe beispielsweise Methyl, Ethyl, Propyl, Isopropyl, n-Butyl, iso-Butyl, sec-Butyl, tert-Butyl, Pentyl, Hexyl, Heptyl, Octyl, 2-Etylhexyl, 2,4,4-Trimethylpentyl, Decyl, 1,1-Dimethylpropyl, 1,1-Dimethylbutyl oder 1,1,3,3-Tetramethylbutyl,
eine gegebenenfalls substituierte C₅- bis C₁₂-Cycloalkylgruppe, Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcydopentyl sowie ein gesättigtes oder ungesättigtes bicyclisches System wie z.B. Norbornyl oder Norbornenyl,
eine gegebenenfalls substituierte C₇ bis C₁₀-Aralkylgruppe beispielsweise Benzyl, 1-Phenylethyl, 2-Phenylethyl, α,α-Dimethylbenzyl, Benzhydryl, p-Tolylmethyl,l-(p-Butylphenyl)-ethyl, o-, m- oder p-Chlorbenzyl, 2,4-Dichlorbenzyl, o-, m- oder p-Methoxybenzyl oder o-, m- oder p-Ethoxybenzyl,
eine gegebenenfalls substituierte C₆-C₁₂-Arylgruppe beispielsweise Phenyl, 2-, 3- oder 4-Methylphenyl, a-Naphthyl oder β-Naphthyl,
gegebenenfalls durch ein oder mehrere Sauerstoff- und/oder Schwefelatome und/oder ein oder mehrere substituierte oder unsubstituierte Iminogruppen unterbrochene oder durch funktionelle Gruppen, Aryl, Alkyl, Aryloxy, Alkyloxy, Halogen, Heteroatome und/oder Heterocyclen substituierte C₁-C₂₀-Alkyl beispielsweise 2-Carboxyethyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Hydroxyethyl, 2-Hydroxypropyl, 3-Hydroxypropyl, 4-Hydroxybutyl, 6-Hydroxyhexyl, 1-Hydroxy-1,1-dimethylmethyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und
gegebenenfalls durch ein oder mehrere Sauerstoff- und/oder Schwefelatome und/oder ein oder mehrere substituierte oder unsubstituierte Iminogruppen unterbrochene oder durch funktionelle Gruppen, Aryl, Alkyl, Aryloxy, Alkyloxy, Halogen, Heteroatome und/oder Heterocyclen substituierte C₆ bis C₁₂-Aryl beispielsweise Tolyl, Xylyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl.

Beispiele für R¹ bis R⁴ sind jeweils unabhängig voneinander Methyl, Ethyl, n-Propyl, Isopropyl, n-Butyl, sec-Butyl, tert.-Butyl, Pentyl, Hexyl, Heptyl, Octyl, 2-Etylhexyl, Phenyl, α- oder β-Naphthyl, Benzyl, Cyclopentyl oder Cyclohexyl.

Bilden zwei oder mehr der Reste R¹ bis R⁴ einen Ring, so können diese beispielsweise 1,4-Butylen, 1,5-Pentylen, 3-Oxa-1,5-pentylen, 3-Aza-1,5-pentylen oder 3-Methyl-3-aza-1,5-pentylen sein.

Bevorzugt für die Reste R¹ bis R⁴ sind unabhängig voneinander Methyl, Ethyl, Propyl, Isopropyl, n-Butyl, tert.-Butyl, Phenyl und Benzyl, besonders bevorzugt sind Methyl, Ethyl, n-Butyl, Phenyl und Benzyl, ganz besonders bevorzugt sind Methyl, Ethyl und n-Butyl und insbesondere Methyl.

Beispiele für R⁵ und R⁶ sind unabhängig voneinander Wasserstoff, Methyl, Ethyl, n-Propyl, n-Butyl, tert.-Butyl, Pentyl, Hexyl, Heptyl, Octyl, 2-Etylhexyl, Phenyl, α- oder β-Naphthyl, Benzyl, Cyclopentyl, Cyclohexyl, Hydroxymethyl, 2-Hydroxyethyl, 2-Carboxyethyl, 2-Methoxycarbonylethyl, 2-Ethoxycarbonylethyl, 2-n-Butoxycarbonylethyl oder 2-Cyanoethyl sein. Bevorzugt sind Wasserstoff, Methyl, Ethyl, n-Propyl, n-Butyl, Phenyl, 2-Carboxyethyl und 2-Hydroxyethyl, besonders bevorzugt sind Wasserstoff, Methyl, Ethyl, n-Butyl und Phenyl, ganz besonders bevorzugt sind Wasserstoff, Methyl, Ethyl und n-Butyl und insbesondere Wasserstoff und Methyl.

Bilden die Reste R⁵ und R⁶ einen Ring, so können diese beispielsweise 1,4-Butylen, oder 1,5-Pentylen sein.

Beispiele für Ammonium-Kationen sind Tetraoctylammonium, Tetramethylammonium, Tetraethylammonium, Tetra-n-butylammonium, Trimethylbenzylammonium, Triethylbenzylammonium, Tri-n-butylbenzylammonium, Trimethylethylammonium, Tri-n-butylethylammonium, Triethylmethylammonium, Tri-n-butylmethylammonium, Di-iso-Propyl-diethylammonium, Di-iso-Propyl-ethyl-methylammonium, Di-iso-Propyl-ethyl-benzylammonium, N,N-Dimethylpiperidinium, N,N-Dimethylmorpholinium, N,N-Dimethylpiperazinium oder N-Methyl-diazabicyclo[2,2,2]octan. Bevorzugte Alkylammoniumionen sind Tetraoctylammonium, Tetramethylammonium, Tetraethylammonium und Tetra-n-butylammonium, besonders bevorzugt sind Tetramethylammonium und Tetraethylammonium und ganz besonders bevorzugt ist Tetramethylammonium.

Ringsysteme enthaltende Ammoniumionen sind beispielsweise methylierte, ethylierte oder benzylierte Piperazine, Piperidine, Morpholine, Chinuclidine oder Triethylendiamine.

Beispiele für α-Hydroxycarboxylate sind beispielsweise Glykolsäure (Hydroxyessigsäure), Milchsäure, Zitronensäure, 2-Methylmilchsäure (α-Hydroxy-iso-buttersäure), 2-Hydroxy-2-methylbuttersäure, 2-Hydroxy-2-ethylbuttersäure, 2-Hydroxy-3-methylbuttersäure, 2-Hydroxycapronsäure, Äpfelsäure, Weinsäure, Glucuronsäure, Gluconsäure, Citramalsäure, Saccharinsäure, Ribonsäure, Benzilsäure, Chinasäure, Mandelsäure, Hexahydromandelsäure, 2-Hydroxycapronsäure oder 3-Phenylmilchsäure. Bevorzugte α-Hydroxycarboxytate sind Milchsäure, 2-Methylmilchsäure (α-Hydroxy-iso-buttersäure), 2-Hydroxy-2-methylbuttersäure und 2-Hydroxycapronsäure, besonders bevorzugt sind Milchsäure, 2-Methylmilchsäure (α-Hydroxy-iso-buttersäure) und 2-Hydroxycapronsäure und ganz besonders bevorzugt ist Milchsäure.

Dabei spielt es bei chiralen Verbindungen erfindungsgemäß keine Rolle, welches Enantiomer oder Diastereomer eingesetzt wird, oder ob die Säuren racemisch eingesetzt werden.

α-Hydroxycarbonsäuren bzw. α-Hydroxycarboxylate im Sinne dieser Schrift sind solche Carbonsäuren bzw. deren Salze, die an einem direkt an eine Carboxylgruppe gebunden Kohlenstoffatom mit genau einer Hydroxygruppe (-OH) substituiert sind. Diese Struktureinheit kann ein- oder mehrmals innerhalb eines Moleküls vorliegen, beispielsweise ein- bis sechsmal, bevorzugt ein- bis viermal, ganz besonders bevorzugt ein- bis dreimal, insbesondere ein- bis zweimal und speziell einmal.

Die erfindungsgemäßen Trimerisierungskatalysatoren sind in der Regel auch bei Temperaturen über 100 °C thermisch beständig und somit über einen Temperaturbereich von ungefähr 30 bis 160 °C katalytisch wirksam, wohingegen Säure-Base-Salze von tertiären Aminen bereits bei Temperaturen um 130 °C (s. US 3,862,150, Bsp. 2) zersetzt werden, wobei die Zersetzungsreaktion bereits bei wesentlich niedrigeren Temperaturen einsetzt, und quartäre Hydroxyalkylammoniumcarboxylate aufgrund ihrer thermischen Labilität in der Regel nur bei Temperaturen von 60 bis 80 °C verwendbar sind.

Höhere Trimerisierungstemperaturen, beispielsweise über 95 °C, werden jedoch häufig zur Trimerisierung von sterisch behinderten Diisocyanaten, wie z.B. Isophorondüsocyanat oder 2-Butyl-2-ethyl-pentandiisocyanat-1,5, und insbesondere zur Herstellung höherer Oligomere angewandt, da dadurch höhere Raum-Zeit-Ausbeuten erreicht werden können. Die Reaktionsgeschwindigkeit der Trimerisierungsreaktion kann bei Verwendung der erfindungsgemäßen tetrasubstituierten Ammonium α-Hydroxycarboxylate im Vergleich zu handelsüblichen Trimerisierungskatalysatoren, wie vorzugsweise N-(2-Hydroxypropyl)-N,N,N-Trimethylammonium-2-ethylhexanoat (DABCO TMR® der Firma Air Products mindestens beibehalten oder sogar erhöht werden. Zudem werden auch Isocyanuratgruppen aufweisende Polyisocyanate mit äußerst niedrigen Farbzahlen nach Hazen (DIN ISO 6271), z.B. vorzugsweise kleiner als 40 (bei HDI), bzw. unter 200, bevorzugt unter 100 (bei IPDI) erhalten.

Wie bereits dargelegt wurde, können die erfindungsgemäß verwendbaren Trimerisierungskatalysatoren nach bekannten Verfahren hergestellt werden. Zur Herstellung von tetrasubstituierten Ammonium α-Hydroxycarboxylaten der Formel (I), vorzugsweise von Tetraalkylammonium α-Hydroxycarboxylaten und besonders bevorzugt von Trialkylmethylammonium α-Hydroxycarboxylate, können tertiäre Amine mit einem Alkylierungsmittel, beispielsweise Alkylhalogeniden, Dialkylcarbonaten oder Dialkylsulfaten, in Abwesenheit oder Gegenwart von Lösungsmitteln, z.B. Chlorbenzol, Toluol oder Xylol, bei Temperaturen von zweckmäßigerweise 100 bis 180 °C zur Reaktion gebracht werden. Gegebenenfalls kann die Reaktion unter Druck durchgeführt werden, wenn das eingesetzte Amin unter den Reaktionsbedingungen gasförmig ist.

Bevorzugte Alkylierungsmittel sind Methylchlorid, Ethylchlorid, Methyliodid, Dimethylcarbonat, Diethylcarbonat, Di-n-butylcarbonat, Dimethylsulfat und Diethylsulfat sowie ferner Benzylchlorid.

Als geeignete tertiäre Amine seien beispielhaft genannt: Trimethylamin, Triethylamin, Tri-n-butylamin, Ethyl-diisopropylamin, N,N'-Dimethyl-piperazin, N-Methoxyphenylpiperazin, N-Methylpiperidin, N-Ethyl-piperidin, Chinuclidin und Trialkylamine, wie z.B.

Trimethyl-, Triethyl- und Tripropylamin und vorzugsweise 1,4-Dimethyl-piperazin, N,N-Dimethylbenzylamin und Triethylendiamin.

Die nach der Alkylierung erhältlichen tetrasubstituierten Ammoniumionen mit dem Alkylierungsmittel als Gegenion, wie beispielsweise Chlorid, lodid, Methylcarbonat oder Methylsulfat, können dann beispielsweise durch Behandeln mit einem Anionentauscher, werden dann in einer bevorzugten Ausführungsform in das tetrasubstituierte Ammoniumhydroxid überführt, das dann anschließend mit der α-Hydroxycarbonsäure umgesetzt werden kann. Die dabei entstehenden äquivalente Mengen von Wasser können entweder im Katalysator belassen werden oder können bevorzugt durch behandeln mit Trocknungsmittel, wie beispielsweise Molsieb oder Zeolith, oder Azeotropdestillation mit einem Schleppmittel, wie beispielsweise Cyclohexan, Benzol oder Toluol, entfernt bzw. abgereichert werden. In der Regel ist ein Wassergehalt im Katalysator unter 0,5 Gew% für den Einsatz in der erfindungsgemäßen Reaktion ausreichend und wird angestrebt.

Die Anwesenheit von Wasser in der Reaktion führt in der Regel durch Hydrolyse der Isocyanate und Decarboxylierung der entstehenden Carbaminsäuren zu Aminen, die wiederum mit Isocyanaten zu schwerlöslichen, unerwünschten Harnstoffen reagieren.

Man kann auch einen direkten Austausch auf einer lonentauschersäule durchführen. Dazu wird ein basisches lonenaustauscherharz (z.B. Amberlyst®-, Dowex®-oder Sephadex®-Typ) mit Kalilauge oder Natronlauge aktiviert und mit der gewünschten α-Hydroxycarbonsäure beladen. Danach wird die Chromatographiesäule mit dem quart. Ammoniumsalz beschickt und eluiert. Das Eluat enthält das gewünschte quart. Ammoniumcarboxylat. Das Lösemittel kann durch Anlegen von Vakuum entfernt werden.

Im Falle der quart. Ammoniumhalogenide lassen sich die Katalysatoren auch durch Kationenaustausch in Lösung in sehr reiner Form erhalten, wenn als Reaktionspartner die den α-Hydroxycarbonsäuren zugrunde liegenden Silbercarboxylate eingesetzt werden.

Die Herstellung der erfindungsgemäßen Katalysatoren kann beispielsweise analog den Arbeitsvorschriften erfolgen, wie in US 5,691,440, Sp. 11, Z. 24-Sp. 12, Z. 41.

Die Alkylierung von tertiären Aminen kann beispielsweise wie folgt ausgeführt werden: das tertiäre Amin wird gegebenenfalls in einem geeigneten Lösungsmittel, beispielsweise einem C₁-C₄-Alkohol, bevorzugt Methanol oder Ethanol, mit dem Alkylierungsmittel in über- oder unterstöchiometrischen oder bevorzugt äquimolaren Mengen, beispielsweise 0,75 -1,25 mol/mol, bevorzugt 0,9 -1,1 mol/mol, bezogen auf das tertiäre Amin gegebenenfalls bei Überdruck für 30 Minuten bis 24 h bei einer Temperatur zwischen Raumtemperatur und 120 °C, gegebenenfalls bei im Reaktionsverlauf ansteigender Temperatur umgesetzt. Nach Beendigung der Reaktion werden die flüchtigen Bestandteile destillativ abgetrennt und gegebenenfalls gewaschen oder umkristallisiert.

Das dabei erhaltene tetrasubstituierte Ammoniumion mit dem Gegenion des Alkylierungsmittels kann dann beispielsweise an einem mit Hydroxidionen beladenen Anionentauscher gegen ein Hydroxidgegenion ausgetauscht werden, wie es beispielsweise in DE-OS 25 27 242, S. 6, unten oder ebda. in den Herstellungsbeispielen 1 und 2 auf S. 13 und 14 beschrieben ist.

Das so erhaltene oder ein kommerziell verfügbares tetrasubstituiertes Ammonium Hydroxid kann dann mit der gewünschten α-Hydroxycarbonsäure zu dem erfindungsgemäßen Katalysator umgesetzt werden. Dazu wird beispielsweise das tetrasubstituierte Ammonium Hydroxid vorgelegt, beispielsweise in einem Lösungsmittel, bevorzugt einem Lösungsmittel, das mit Wasser ein Azeotrop bildet, beispielsweise einem C₁-C₄-Alkohol, bevorzugt Methanol oder Ethanol, und dazu die gewünschte α-Hydroxycarbonsäure, gegebenenfalls ebenfalls in dem gleichen oder einem anderen Lösungsmittel, langsam zugegeben. Die Zugabe kann 0 bis 100 °C, bevorzugt 0 bis 80, besonders bevorzugt 0 bis 60, ganz besonders bevorzugt 10 bis 40 °C und insbesondere bei Raumtemperatur erfolgen. Nach Abtrennung des gegebenenfalls vorhandenen Lösungsmittels mit gebildetem Reaktionswasser, beispielsweise durch Destillation, gegebenenfalls im Vakuum, ist der erfindungsgemäße Katalysator verwendbar und kann gegebenenfalls in einem Lösungsmittel aufgenommen werden. Ein solches Lösungsmittel kann auch gegenüber Isocyanat reaktive Gruppen enthalten.

Der erfindungsgemäße Katalysator kann in Substanz, als Lösung oder als Suspension eingesetzt werden.

Wird der Katalysator als Lösung eingesetzt, so wird, je nach Löslichkeit im verwendeten Lösungsmittel in der Regel eine 10 - 80 %ige, bevorzugt 10 - 50, besonders bevorzugt 15 - 45 und ganz besonders bevorzugt 30 - 40 Gew%ige Lösung eingestellt.

Als Trimerisierungskatalysatoren können auch Gemische mit anderen bekannten Trimerisierungskatalysatoren eingesetzt werden, wobei diese in breiten Mengenverhältnissen, z.B. in Mengenverhältnissen von 90 : 10 bis 10 : 90, bevorzugt 80:20 bis 20:80 und besonders bevorzugt 60 : 40 bis 40 : 60 gemischt werden können.

Zur Herstellung der Isocyanuratgruppen aufweisenden Polyisocyanate werden die erfindungsgemäßen Trimerisierungskatalysatoren in Abhängigkeit von ihrer katalytischen Wirksamkeit zweckmäßigerweise in möglichst kleinen wirksamen Mengen, die experimentell auf einfache Weise ermittelt werden können, verwendet.

Im allgemeinen gelangen beim erfindungsgemäßen Verfahren die tetrasubstituierten Ammonium α-Hydroxycarboxylate (I) in einer Menge von 0,002 bis 0,05 Gew.%, vorzugsweise von 0,005 bis 0,02 Gew.-% bezogen auf das Gewicht der (cyclo)aliphatischen Diisocyanate, zum Einsatz.

Das erfindungsgemäße Verfahren wird zweckmäßigerweise bei einer Temperatur im Bereich von 10 bis 150°C und Reaktionszeiten von 10 min - 6 Stunden, bevorzugt von 20 min bis 3 Stunden, besonders bevorzugt von 20 min bis 2 Stunden durchgeführt. Bei Temperaturen über 150 °C kann eine Verfärbung der Isocyanuratgruppen aufweisenden Polyisocyanate auftreten, z.B. bei längeren Reaktionszeiten.

Bei Verwendung der erfindungsgemäßen tetrasubstituierten Ammonium α-Hydroxycarboxylaten werden vorzugsweise Reaktionstemperaturen über 50 °C, besonders bevorzugt von 60 bis 120 °C angewandt und im wesentlichen farblose Trimerisierungsprodukte erhalten.

Die Trimerisierung kann kontinuierlich, halbkontinuierlich oder diskontinuierlich durchgeführt werden, bevorzugt diskontinuierlich.

In der Regel spielt es keine Rolle welche Komponenten vorgelegt oder zugegeben werden. Zumeist wird das zu trimerisierende Isocyanat zumindest teilweise, bevorzugt vollständig, vorgelegt und der mindestens eine Katalysator langsam und/oder portionsweise zugegeben, dann auf die gewünschte Reaktionstemperatur gebracht und der Rest des Katalysators, gegebenenfalls portionsweise, zugegeben.

Eine alternative Herstellungsvariante verläuft wie folgt: Beim diskontinuierlichen Verfahren wird in einem Rührreaktor gearbeitet. Dabei wird die Mischung aus Diisocyanat und Katalysator üblicherweise bei ca. 40°C vorgelegt im Anschluss wird die Temperatur der Reaktionsmischung zur Initiierung der Trimerisierung auf 50 bis 140 °C, bevorzugt auf 55 bis 100 °C, erhöht. Alternativ kann der Katalysator auch zudosiert werden, nachdem das Diisocyanat die zur Reaktion notwendige Temperatur erreicht hat. Die Trimerisierung ist in der Regel exotherm, der Katalysator kann in reiner Form eingesetzt werden. Es ist auch möglich, den Katalysator in einem geeignetem Lösemittel zu lösen und in dieser Form zum Einsatz zu bringen.

Die kontinuierliche Trimerisierung wird zweckmäßigerweise in einer Reaktionsschlange unter kontinuierlicher, gleichzeitiger Zudosierung von Diisocyanat und des Katalysators bei 50 bis 160 °C und innerhalb von 30 Sekunden bis 4 Stunden durchgeführt. Eine Reaktionsschlange mit kleinem Durchmesser führt zur Erreichung hoher Stömungsgeschwindigkeiten und folglich guter Durchmischung. Weiterhin ist es vorteilhaft, das Diisocyanat / Katalysator-Gemisch vor Eintritt in die Reaktionsschlange auf ca. 50 bis 60°C zu erhitzen. Zur exakteren Dosierung und optimalen Durchmischung des Katalysators ist es weiterhin vorteilhaft, den Katalysator in einem geeignetem Lösemittel zu lösen. Geeignet sind prinzipiell solche Lösemittel, in denen der Katalysator eine gute Löslichkeit hat. Die kontinuierlich Trimerisierung kann auch in einer Kesselkaskade durchgeführt werden. Denkbar ist auch eine Kombination aus Kesselkaskade und Rohrreaktor.

Üblicherweise wird die Reaktion in einem unter den Reaktionsbedingungen inerten Gas oder Gasgemisch durchgeführt, beispielsweise solche mit einem Sauerstoffgehalt unter 2, bevorzugt unter 1, besonders bevorzugt unter 0,5 Vol%, bevorzugt sind Stickstoff, Argon, Helium, Stickstoff - Edelgas - Gemische, besonders bevorzugt ist Stickstoff.

Nach Erreichen des gewünschten Trimerisierungsgrads bzw. NCO-Gehalts oder Umsetzungsgrades (bezogen auf den NCO-Gehalt vor der Reaktion) der Isocyanurat/(cyclo)aliphatische Diisocyanat-Reaktionsmischung, wobei der Umsatzgrad zweckmäßigerweise im Bereich von 20 bis 45 % der NCO-Gruppen, vorzugsweise von 25 bis 35 % der NCO-Gruppen, liegt und wozu üblicherweise Reaktionszeiten von 0,05 bis 4 Stunden, vorzugsweise von 10 min bis 3 Stunden, erforderlich sind, kann die Trimerisierungsreaktion beispielsweise durch eine Desaktivierung des Trimerisierungskatalysators beendet werden.

Das Produkt enthält neben monomerem Isocyanat Verbindungen, die einen oder auch mehrere Isocyanuratstrukturen aufweisen. Verbindungen dieser Art sind in der Literatur beschrieben.

Als Desaktivierungsmittel eignen sich beispielsweise anorganische Säuren, wie z.B. Chlorwasserstoff, phosphorige Säure oder Phosphorsäure, Carbonsäurehalogenide, wie z.B. Acetylchlorid oder Benzoylchlorid, Sulfonsäuren oder -ester, wie z.B. Methansulfonsäure, p-Toluolsulfonsäure, p-Toluolsulfonsäuremethyl- oder -ethylester, m-Chlorperbenzoesäure und vorzugsweise Dialkylphosphate wie z.B. Di-2-ethylhexylphosphat und insbesondere Dibutylphosphat.

Die Desaktivierungsmittel können, bezogen auf die Trimerisierungskatalysatoren, in äquivalenten oder überschüssigen Mengen verwendet werden, wobei die kleinste wirksame Menge, die experimentell ermittelt werden kann, schon aus wirtschaftlichen Gründen bevorzugt wird. Beispielsweise wird das Desaktivierungsmittel im Verhältnis zum Trimerisierungskatalysator von 1 - 2,5 : 1 mol/mol, bevorzugt 1 - 2 : 1, besonders bevorzugt 1 -1,5 : 1 und ganz besonders bevorzugt 1 -1,2 : 1 mol/mol eingesetzt.

Die Zugabe ist abhängig von der Art des Desaktivierungsmittels. So wird Chlorwasserstoff bevorzugt gasförmig über oder bevorzugt durch das Reaktionsgemisch hindurch geleitet, flüssige Desaktivierungsmittel werden zumeist in Substanz oder als Lösung in einem unter den Reaktionsbedingungen inerten Lösungsmittel und feste Desaktivierungsmittel in Substanz oder als Lösung oder Suspension in einem unter den Reaktionsbedingungen inerten Lösungsmittel zugegeben.

Die Zugabe des Desaktivierungsmittels erfolgt in der Regel bei der Reaktionstemperatur, kann aber auch bei niedrigerer Temperatur erfolgen.

Das erfindungsgemäße Verfahren wird vorzugsweise lösungsmittelfrei durchgeführt. Werden die (cyclo)aliphatischen Diisocyanate jedoch in Gegenwart von Lösungs- oder Verdünnungsmitteln partiell trimerisiert, so eignen sich hierfür sowohl inerte unpolare als auch inerte polare Lösungs- oder Verdünnungsmittel, wie z.B. Toluol, Xylol, cyclische Ether, Carbonsäureester und Ketone oder ihre Gemische.

Die nach dem erfindungsgemäßen Verfahren hergestellten Isocyanuratgruppen aufweisende Polyisocyanate können in an sich bekannter Weise, beispielsweise durch Dünnschichtdestillation bei einer Temperatur von 100 bis 180 °C, gegebenenfalls im Vakuum, gegebenenfalls zusätzlich mit Durchleiten von inertem Strippgas, oder Extraktion von gegebenenfalls vorhandenen Lösungs- oder Verdünnungsmittel und/oder vorzugsweise von überschüssigen, nicht-umgesetzten (cyclo)aliphatischen Diisocyanaten befreit werden, so daß die Isocyanuratgruppen aufweisenden Polyisocyanate mit einem Gehalt an monomeren Diisocyanaten von z.B. unter 1,0 Gew.%, vorzugsweise unter 0,5 Gew.-%, besonders bevorzugt unter 0,3, ganz besonders bevorzugt unter 0,2 und insbesondere nicht mehr als 0,1 Gew% erhältlich sind.

Ohne Abtrennung der überschüssigen monomeren Diisocyanate eignen sich die Isocyanuratgruppen aufweisende Polyisocyanate beispielsweise zur Herstellung von PU-Schaumstoffen, zelligen oder kompakten Elastomeren, Vergußmassen und Klebstoffen. Die monomerfreien und monomerhaltigen Isocyanuratgruppen aufweisenden Polyisocyanaten können ferner in an sich bekannter Weise durch Einführung von z.B. Urethan-, Allophanat-, Harnstoff-, Biuret- und/oder Carbodiimidgruppen modifiziert und/oder die Isocyanatgruppen mit geeigneten Blockierungsmitteln, wie z.B. ε-Caprolactam, Malonsäuredimethylester, Acetessigester oder aromatischen Hydroxylgruppen blockiert werden.

Nach dem erfindungsgemäßen Verfahren können beliebige, organische Diisocyanate mit aliphatischen, cycloaliphatischen oder aliphatischen und cycloaliphatischen Isocyanatgruppen oder ihre Gemische trimerisiert werden.

Geeignete aliphatische Diisocyanate besitzen vorteilhafterweise 3 bis 16 C-Atome, vorzugsweise 4 bis 12 C-Atome im linearen oder verzweigtkettigen Alkylenrest und geeignete cycloaliphatische Diisocyanate vorteilhafterweise 4 bis 18 C-Atome, vorzugsweise 6 bis 15 C-Atome im Cycloalkylenrest. Beispielhaft genannt seien: 1,4-Diisocyanato-butan, 2-Ethyl-1,4-diisocyanato-butan, 1,5-Diisocyanato-pentan, 2-Methyl-1,5-diisocyanato-pentan, 2,2-Dimethyl-1 ,5-diisocyanato-pentan, 2-Propyl-2-ethyl-1,5-diisocyanato-pentan, 2-Butyl-2-ethyl-1,5--disocyanato-pentan, 2-Alkoxymethylen-1,5-diisocyanato-pentan, 3-Methyl-, 3-Ethyl-1,5-diisocyanato-pentan, 1,6-Hexamethylen-diisocyanat, 2,4,4- bzw. 2,2,4-Trimethyl-1,6-hexamethylen-diisocyanat, 1,7-Diisocyanatoheptan, 1,8-Diisocyanato-octan, 1,10-Diisocyanato-decan, 1,12-Diiso-cyanato-dodecan, 4,4'-Diisocyanato-dicyclohexylmethan, 2,4'-Diisocyanato-dicyclohexylmethan sowie Gemische der Diisocyanato-dicyclohexylmethan-Isomeren, 1,3-Diisocyanato-cyclohexan sowie Isomerengemische von Diisocyanato-cyclohexanen und 1-Isocyanato-3-isocyanatomethyl-3,5,5-trimethylcyclohexan. Als (cyclo)aliphatische Diisocyanate vorzugsweise verwendet werden 1,6-Hexamethylen-diisocyanat, isomere aliphatische Diisocyanate mit 6 Kohlenstoffatomen im Alkylenrest sowie Mischungen davon, 2-Butyl-2-ethyl-1,5-diisocyanato-pentan und 1-Isocyanato-3-isocyanatomethyl-3,5,5-trimethylcyclohexan, besonders bevorzugt sind 1,6-Hexamethylen-diisocyanat und 1-Isocyanato-3-isocyanatomethyl-3,5,5-trimethylcyclohexan sowie Mischungen davon, beispielsweise im Verhältnis 10:90 - 90:10, bevorzugt 20:80 - 80:20 und besonders bevorzugt 33:67 - 67:33.

Selbstverständlich katalysieren die erfindungsgemäßen Katalysatoren auch die Trimerisierung aromatischer Isocyanate, sind jedoch für (cyclo)aliphatische Isocyanate bevorzugt.

Die erfindungsgemäßen neuen Trimerisierungskatalysatoren können zur Trimerisierung von nach beliebigen Verfahren, beispielsweise gemäß einem phosgenfreien oder einem unter Verwendung von Phosgen ablaufenden Verfahrensweg, hergestellten (cyclo)aliphatischen Diisocyanaten verwendet werden.

Die erfindungsgemäß verwendbaren (cyclo)aliphatischen Diisocyanate können nach beliebigen Verfahren, beispielsweise durch Phosgenierung der entsprechenden Diamine und thermische Spaltung der intermediär gebildeten Dicarbaminsäurechloride hergestellt werden. Nach phosgenfreien Verfahren hergestellte (cyclo)aliphatische Diisocyanate enthalten als Nebenprodukte keine Chlorverbindungen und besitzen daher herstellungsbedingt ein grundsätzlich anderes Nebenproduktspektrum.

Selbstverständlich können auch Gemische von Isocyanaten, die nach dem Phosgenverfahren und nach phosgenfreien Verfahren hergestellt sind, eingesetzt werden.

Es zeigte sich, daß die erfindungsgemäß verwendbaren Trimerisierungskatalysatoren bei der Trimerisierung auch von nach dem Phosgenverfahren hergestellten (cyclo)aliphatischen Diisocyanaten eine gute katalytische Wirksamkeit aufweisen und Isocyanuratgruppen aufweisende Polyisocyanate mit niedriger Farbzahl ergeben.

Die in das erfindungsgemäße Verfahren einsetzbaren nach einem phosgenfreien Verfahren und insbesondere durch thermische Spaltung von (cyclo)aliphatischen Dicarbaminsäureestern erhältliche (cyclo)aliphatische Diisocyanate sind nicht beschränkt, wobei durch thermische Spaltung von (cyclo)aliphatischen Dicarbaminsäureestern erhältliche Diisocyanate aus der Gruppe 1,6-Hexamethylen-diisocyanat, 2-Butyl-2-ethyl-pentamethylen-diisocyanat-1,5 und 1-Isocyanato-3-isocyanatomethyl-3,5,5-trimethylcyclohexan insbesondere bevorzugt verwendet werden.

In einer bevorzugt Ausführungsform der Erfindung werden Isocyanate eingesetzt, die einen Gesamtchlorgehalt von 100 Gew.ppm oder weniger aufweisen, bevorzugt 80 Gew.-ppm oder weniger.

Nach diesen Verfahrensvarianten hergestellte Isocyanuratgruppen aufweisende Polyisocyanate eignen sich vorzüglich zur Herstellung von Polyurethan-Beschichtungen, z.B. von Textil- und Lederbeschichtungen, für -Dispersionen und -Klebstoffe und finden insbesondere Verwendung als Polyisocyanatkomponente in Ein- und Zweikomponenten-Polyurethansystemen für hochwertige, wetterbeständige Polyurethan-Lacke und high-solid-Lacke.

In dieser Schrift verwendete ppm- und Prozentangaben beziehen sich, falls nicht anders angegeben, auf Gewichtsprozente und -ppm.

Die folgenden Beispiele sollen die Erfindung erläutern, aber nicht auf diese Beispiele einschränken.

### Beispiele

Die Herstellung der tetrasubstituierten α-Hydroxycarboxylate erfolgte durch Umsetzung der α-Hydroxycarbonsäure mit einem tetrasubstituierten Ammoniumhydroxid in methanolischer Lösung. Die methanolische Carbonsäurelösung wurde vorgelegt und das tetrasubstituierte Ammoniumhydroxid, gelöst in Methanol, wurde innerhalb 30 min zugetropft. Nach vollständiger Zugabe des Ammoniumhydroxids wurde bei ca. 40°C 1 Stunde nachgerührt. Anschließend wurden im Rotationsverdampfer entstandenes Reaktionswasser und andere flüchtige Bestandteile entfernt. Der farblose Rückstand wurde in geeigneten Lösungsmitteln, wie Ethyldiglycol oder Ethylenglycol gelöst. Es kann das Carboxylat auch aus geeigneten Lösungsmitteln, wie Essigester umkristallisiert werden.

### Trimerisation von HDI, allgemeine Vorschrift zur Trimerisierung von Isocyanaten

Für die nachstehenden Versuche wurde halogenfreies Hexamethylendiisocyanat (HDI) verwendet, wenn nicht anders angegeben.

### Vergleichsbeispiel 1

100 g Hexamethylendiisocyanat wurden bei 80°C unter Stickstoffabdeckung vorgelegt und unter Rühren innerhalb von ca. 30 min 50 ppm DABCO- TMR (DABCO-TMR = N-(2-Hydroxypropyl)-N,N,N-Trimethylammonium-2-ethylhexanoat) zugegeben und 20 min nachgerührt. Der NCO-Wert fiel auf 37,2%. Die Reaktion wurde dann mit Phoshorsäure-bis-(2-ethylhexylester) und das Reaktionsprodukt in einem Dünnschichtverdampfer bei 140°C entgast.

Nach der Entgasung wurde eine Farbzahl von 53 Hz gemessen, das Produkt hatte ein NCO-Wert von 20,9%

### Beispiel 1

100 g Hexamethylendiisocyanat wurde wie unter Vergleichsbeispiel 1 verarbeitet, jedoch wurde als Trimerisierungskatalysator das Carboxylat aus Tetramethylammoniumhydroxid mit 2-Hydroxypropionsäure verwendet. Nach dem Zusatz von 50 ppm dieses Katalysators und einer Gesamtreaktionszeit von 35 min wurde ein NCO-Wert von 27% gemessen. Die Reaktion wurde dann mit Phoshorsäure-bis-(2-ethylhexylester) abgebrochen.

Nach der destillativen Ausarbeitung lag der NCO-Wert bei 19,0 %. Es wurde ein sehr helles Produkt mit einer Farbzahl von 30 Hz erhalten.

### Beispiel 2

100 g Hexamethylendiisocyanat wurde wie unter Vergleichsbeispiel 1 verarbeitet, jedoch wurde als Trimerisierungskatalysator das Carboxylat aus Tetramethylammoniumhydroxid mit 2-Ethyl-2-Hydroxybuttersäure verwendet. Nach dem Zusatz von 50 ppm dieses Katalysators und einer Gesamtreaktionszeit von 50 min wurde ein NCO-Wert von 39,0% gemessen. Die Reaktion wurde dann mit Diethylhexylphosphat abgebrochen.

Nach der destillativen Ausarbeitung lag der NCO-Wert bei 21,2 %. Es wurde ein sehr helles Produkt mit einer Farbzahl von 21 Hz erhalten.

### Beispiel 3

100 g Hexamethylendiisocyanat wurde wie unter Vergleichsbeispiel 1 verarbeitet, jedoch wurde als Trimerisierungskatalysator das Carboxylat aus Tetramethylammoniumhydroxid mit 2-Hydroxyisocapronsäure verwendet. Nach dem Zusatz von 50 ppm dieses Katalysators und einer Gesamtreaktionszeit von 35 min wurde ein NCO-Wert von 27% gemessen. Die Reaktion wurde dann mit Phoshorsäure-bis-(2-ethylhexylester) abgebrochen.

Nach der destillativen Ausarbeitung lag der NCO-Wert bei 20,7 %. Es wurde ein sehr helles Produkt mit einer Farbzahl von 30 Hz erhalten.

### Beispiel 4

Es wurde HDI aus einem Phosgenprozess verwendet.

100 g Hexamethylendiisocyanat wurde wie unter Vergleichsbeispiel 1 verarbeitet, jedoch wurde als Trimerisierungskatalysator das Carboxylat aus Tetramethylammoniumhydroxid mit 2-Ethyl-2-hydroxybuttersäure verwendet. Nach dem Zusatz von 35 ppm dieses Katalysators und einer Gesamtreaktionszeit von 40 min wurde ein NCO-Wert von 26,5% gemessen. Die Reaktion wurde dann mit Phoshorsäure-bis-(2-ethylhexylester) abgebrochen.

Nach der destillativen Ausarbeitung lag der NCO-Wert bei 21,2 %. Es wurde ein sehr helles Produkt mit einer Farbzahl von 17 Hz erhalten.

### Vergleichsbeispiel 2 :

100 g Hexamethylendiisocyanat aus einem Phosgenprozeß mit einem Gesamtchlorgehalt vom 25 ppm wurden bei 80°C unter Stickstoffabdeckung vorgelegt und unter Rühren innerhalb von ca. 30 min 50 ppm DABCO- TMR (DABCO-TMR = N-(2-Hydroxypropyl)-N,N,N-Trimethylammonium-2-ethylhexanoat) zugegeben und 20 min nachgerührt. Der NCO-Wert fiel auf 36,8%. Die Reaktion wurde dann mit Phoshorsäure-bis-(2-ethylhexylester) und das Reaktionsprodukt in einem Dünnschichtverdampfer bei 140°C entgast.

Nach der Entgasung wurde eine Farbzahl von 61 Hz gemessen, das Produkt hatte ein NCO-Wert von 21,5%.

### Trimerisierung von IPDI

### Vergleichsbeispiel 3

750 g frisch destilliertes Isophorondiisocyanat wurden vorgelegt und unter N₂-Zufuhr auf 80°C aufgeheizt. Unter gleichzeitiger Stickstoffeinleitung wurde portionsweise DACBO TMR® zugegeben. Nach dem Zusatz von 900 ppm dieses Katalysators und einer Gesamtreaktionszeit von 2 Stunden wurde ein NCO-Wert von 32% in der Reaktionslösung gemessen. Die Reaktion wurde dann mit Phoshorsäure-bis-(2-ethylhexylester) abgebrochen.

Nach der destillativen Ausarbeitung lag der NCO-Wert bei 17,2 %. Es wurde ein stark gefärbtes Produkt mit einer Farbzahl von 800 Hz erhalten. (gemessen 70%ig in Butylacetat)

### Beispiel 5

750 g frisch destilliertes Isophorondiisocyanat wurden vorgelegt und auf 80°C aufgeheizt. Unter gleichzeitiger Stickstoffzufuhr wurde portionsweise 160 ppm des Carboxylates aus Tetramethylammoniumhydroxid mit 2-Ethyl-2-hydroxybuttersäure zugegeben. Nach einer Gesamtreaktionszeit von 2 Stunden wurde ein NCO-Wert von 32,8% in der Reaktionslösung gemessen. Die Reaktion wurde dann mit Phoshorsäure-bis-(2-ethylhexylester) abgebrochen.

Nach der destillativen Ausarbeitung lag der NCO-Wert bei 17,1 %. Es wurde ein leicht gelblich gefärbtes Produkt mit einer Farbzahl von 180 Hz erhalten. (gemessen 70%ig in Butylacetat)

### Beispiel 6

750 g frisch destilliertes Isophorondiisocyanat wurden vorgelegt und auf 80°C aufgeheizt. Unter gleichzeitiger Stickstoffzufuhr wurde portionsweise 160 ppm des Carboxylates aus Tetramethylammoniumhydroxid mit 2-Hydroxypropionsäure zugegeben. Nach einer Gesamtreaktionszeit von 2 Stunden wurde ein NCO-Wert von 26,1% in der Reaktionslösung gemessen. Die Reaktion wurde dann mit Phoshorsäure-bis-(2-ethylhexylester) abgebrochen.

Nach der destillativen Ausarbeitung lag der NCO-Wert bei 17,3 %. Es wurde ein helles Produkt mit einer Farbzahl von 92 Hz erhalten. (gemessen 70%ig in Butylacetat)

## Patentansprüche

1. Verfahren zur Herstellung von Isocyanuratgruppen-enthaltenden Polyisocyanaten durch zumindest teilweise Trimerisierung von (cyclo)aliphatischen Diisocyanaten, **dadurch gekennzeichnet, daß** man die Umsetzung in Gegenwart mindestens eines Trimerisierungskatalysators, ausgewählt aus der Gruppe der mit vier Kohlenwasserstoffresten substituierten Ammoniumsalze von α-Hydroxycarboxylaten, durchführt.

2. Verfahren zur Herstellung von Isocyanuratgruppen-enthaltenden Polyiscoyansten durch zumindest teilweise Trimerisierung von (cyclo) aliphatischen Diisocyanaten, **dadurch gekennzeichnet, daß** man als Trimerisierungskatalysator mindestens eine Verbindung der Formel (I) einsetzt, 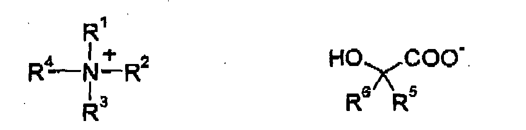 worin
R¹, R², R³, R⁴, R⁵ und R⁶ unabhängig voneinander jeweils gleich oder verschieden sein können und für eine geradkettige oder verzweigte C₁- bis C₂₀-Alkylgruppe, eine gegebenenfalls substituierte C₅- bis C₁₂-Cycloalkylgruppe, eine gegebenenfalls substituierte C₇- bis C₁₀-Aralkylgruppe, oder eine gegebenenfalls substituierte C₆-C₁₂-Arylgruppe stehen oder
zwei oder mehr der Reste R¹ bis R⁴ gemeinsam miteinander eine 4-, 5- oder 6-gliedrige Alkylenkette bilden oder zusammen mit einem Stickstoffatom einen 5-oder 6-gliedrigen Ring bilden, der noch ein zusätzliches Stickstoff- oder Sauerstoffatom als Brückenglied enthalten kann oder gemeinsam ein mehrgliedriges, vorzugsweise sechsgliedriges Mehrringsystem, vorzugsweise Zweiringsystem bilden, das noch ein oder mehrere zusätzliche Stickstoffatome, Sauerstoffatome oder Stickstoff- und Sauerstoffatome als Brückenglieder enthalten kann und
R⁵ und R⁶ zusätzlich Wasserstoff, gegebenenfalls durch ein oder mehrere Sauerstoff- und/oder Schwefelatome und/oder ein oder mehrere substituierte oder unsubstituierte iminogruppen unterbrochene oder durch funktionelle Gruppen, Aryl, Alkyl, Aryloxy, Alkyloxy, Halogen, Heteroatome und/oder Heterocyclen substituierte C₁-C₂₀-Alkyl oder C₆ bis C₁₂-Aryl
sein können.

3. Verfahren nach Anspruch 2, **dadurch gekennzeichnet, daß** die Reste R¹ bis R⁴ unabhängig voneinander ausgewählt sind aus der Gruppe bestehend aus Methyl, Ethyl, Propyl, Isopropyl, n-Bulyl, tert.-Butyl, Phenyl und Benzyl.

4. Verfahren nach Anspruch 2 oder 3, **dadurchgekennzeichnet**, daß die Reste R⁵ und R⁶ unabhängig voneinander ausgewählt sind aus der Gruppe bestehend aus Wasserstoff, Methyl, Ethyl, n-Propyl, n-Butyl, Phenyl, 2-Carboxyethyl und 2-Hydroxyethyl.

5. Verfahren nach Anspruch 1 oder 2, **dadurch gekennzeichnet, daß** das Ammonium-lon ausgewählt ist aus der Gruppe bestehend aus Tetraoctylammonium, Tetramethylammonium, Tetraethylammonium, Tetra-n-butylammonium, Trimethylbenzylammaniüm, Triethylbenzylammonium, Tri-n-butylbenzylammonium, Trimethylethylammonium, Tri-n-butylethylammonium, Triethylmethylammonium, Tri-n-butylmethylammonium, Di-iso-Propyl-diethylammonium, Di-iso-Propyl-ethyl-methylammonium, Di-iso-Propyl-ethyl-benzylammonium, N,N-Dimethylpiperidinium, N,N-Dimethylmorpholinium, N,N-Dimethylpiperazinium oder N-Methyldiazabicyclo[2,2,2]octan.

6. Verfahren nach Anspruch 1 oder 2, **dadurch gekennzeichnet, daß** das α-Hydroxycarboxylat-lon ausgewählt ist aus der Gruppe bestehend aus den Anionen der Glykolsäure (Hydroxyessigsäure), Milchsäure, Zitronensäure, 2-Methylmilchsäure (α-Hydroxy-iso-buttersäure), 2-Hydroxy-2-methylbuttersäure, 2-Hydroxy-2-ethylbuttersäure, 2-Hydroxy-3-methylbuttersäure, 2-Hydroxycapronsäure, Äpfelsäure, Weinsäure, Glucuronsäure, Glukonsäure, Citramalsäure, Saccharinsäure, Ribonsäure, Benzilsäure, Chinasäure, Mandelsäure, Hexahydromandelsäure, 2-Hydroxycapronsäure und 3-Phenyimilchsäure.

7. Verfahren nach einem der vorstehenden Ansprüche, **dadurch gekennzeichnet, daß** man nach Erreichen des angestrebten Trimerisierungsgrads den Trimerisierungskatalysator desaktiviert.

8. Verfahren nach Anspruch 7, **dadurch gekennzeichnet, daß** man den Trimerisierungskatalysator mit Dibutylphosphat oder Di-(2-ethylhexyl) phosphat desaktiviert.

9. Verfahren nach einem der vorstehenden Ansprüche, **dadurch gekennzeichnet, daß** man als Diisocyanate solche einsetzt, die einen Gesamtchlorgehalt von weniger als 100 Gew.-ppm aufweisen.

10. Verfahren nach einem der vorstehenden Ansprüche, **dadurch gekennzeichnet, daß** man als Diisocyanate 1,6-Hexamethylen-diisocyanat und/oder 1-lsocyanato-3-isocyanatomethyl-3,5,5-trimethylcyclohexan einsetzt.

11. Verwendung von mit vier Kohlenwasserstoffresten substituierten Ammoniumsalzen von α-Hydroxycarboxylaten als Trimerisierungskatalysator für Isocyanate.

12. Verfahren zur Herstellung von Polyurethan-Beschichtungen, Polyurethan-Dispersionen, Polyurethan-Klebstoffen und Polyurethan-Lacken, in dem man ein lsocyanuratgruppen-enthaltendes Polyisocyanat durch zumindest teilweise Trimerisierung von (cyclo)aliphatischen Diisocyanaten herstellt und dieses Polyisocyanat als Polyisocyanatkomponente in Ein- oder Zweikomponenten-Polyurethansystemen einsetzt, **dadurch gekennzeichnet, daß** man die Umsetzung vom (cyclo)aliphatischen Diisocyanat zum Isocyanuratgruppen-enthaltenden Polyisocyanat in Gegenwart mindestens eines Trimerisierungskatalysators, ausgewählt aus der Gruppe der mit vier Kohlenwasserstoffresten substituierten Ammoniumsalze von α-Hydroxycarboxylaten, gemäß einem der Ansprüche 1 bis 10 durchführt.

## Claims

1. A process for preparing isocyanurate-comprising polyisocyanates by at least partly trimerizing (cyclo) aliphatic diisocyanates, which comprises carrying out the reaction in the presence of at least one trimerization catalyst selected from the group of the ammonium salts, substituted by four hydrocarbon radicals, of α-hydroxycarboxylates.

2. A process for preparing isocyanurate-comprising polyisocyanates by at least partly trimerizing (cyclo) aliphatic diisocyanates, which comprises using, as the trimerization catalyst, at least one compound of the formula (I) 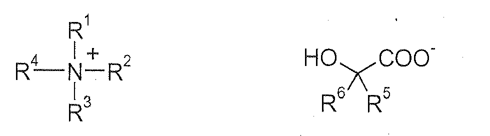 where
R¹, R², R³, R⁴, R⁵ and R⁶ may each independently be the same or different and are each a straight-chain or branched C₁- to C₂₀-alkyl group, an optionally substituted C₅- to C₁₂-cycloalkyl group, an optionally substituted C₇- to C₁₀-aralkyl group, or an optionally substituted C₆-C₁₂-aryl group, or two or more of the R¹ to R⁴ radicals together form a 4-, 5- or 6-membered alkylene chain or, together with a nitrogen atom, form a 5- or 6-membered ring which may also comprise an additional nitrogen or oxygen atom as a bridge member, or together form a multimembered, preferably six-membered, polycyclic system, preferably bicyclic system, which may also comprise one or more additional nitrogen atoms, oxygen atoms or oxygen and nitrogen atoms as bridge members, and
R⁵ and R⁶ may additionally be hydrogen, or C₁-C₂₀-alkyl or C₆- to C₁₂-aryl, each optionally interrupted by one or more oxygen and/or sulfur atoms and/or one or more substituted or unsubstituted imino groups, or substituted by functional groups, aryl, alkyl, aryloxy, alkyloxy, halogen, heteroatoms and/or heterocycles.

3. The process according to claim 2, wherein the R¹ to R⁴ radicals are each independently selected from the group consisting of methyl, ethyl, propyl, isopropyl, n-butyl, tert-butyl, phenyl and benzyl.

4. The process according to claim 2 or 3, wherein the R⁵ and R⁶ radicals are each independently selected from the group consisting of hydrogen, methyl, ethyl, n-propyl, n-butyl, phenyl, 2-carboxyethyl and 2-hydroxyethyl.

5. The process according to claim 1 or 2, wherein the ammonium ion is selected from the group consisting of tetraoctylammonium, tetramethylammonium, tetraethylammonium, tetra-n-butylammonium, trimethylbenzylammonium, triethylbenzylammonium, tri-n-butylbenzylammonium, trimethylethylammonium, tri-n-butylethylammonium, triethylmethylammonium, tri-n-butylmethylammonium, diisopropyldiethylammonium, diisopropylethylmethylammonium, diisopropylethylbenzylammonium, N,N-dimethylpiperidinium, N,N-dimethylmorpholinium, N,N-dimethylpiperazinium or N-methyldiazabicyclo[2.2.2]octane.

6. The process according to claim 1 or 2, wherein the α-hydroxycarboxylate ion is selected from the group consisting of the anions of glycolic acid (hydroxyacetic acid), lactic acid, citric acid, 2-methyllactic acid (α-hydroxyisobutyric acid), 2-hydroxy-2-methylbutyric acid, 2-hydroxy-2-ethylbutyric acid, 2-hydroxy-3-methylbutyric acid, 2-hydroxycaproic acid, malic acid, tartaric acid, glucuronic acid, gluconic acid, citramalic acid, saccharic acid, ribonic acid, benzilic acid, quinic acid, mandelic acid, hexahydromandelic acid, 2-hydroxycaproic acid and 3-phenyllactic acid.

7. The process according to any of the preceding claims, wherein the trimerization catalyst is deactivated after the desired degree of trimerization has been attained.

8. The process according to claim 7, wherein the trimerization catalyst is deactivated with dibutyl phosphate or di(2-ethylhexyl) phosphate.

9. The process according to any of the preceding claims, wherein the diisocyanates used are those which have a total chlorine content of less than 100 ppm by weight.

10. The process according to any of the preceding claims, wherein the diisocyanates used are hexamethylene 1,6-diisocyanate and/or 1-isocyanato-3-isocyanatomethyl-3,5,5-trimethylcyclohexane.

11. The use of ammonium salts, substituted by four hydrocarbon radicals, of a-hydroxycarboxylates as a trimerization catalyst for isocyanates.

12. A process for producing polyurethane coatings, polyurethane dispersions, polyurethane adhesives and polyurethane lacquers, in which an isocyanurate-comprising polyisocyanate is prepared by at least partial trimerization of (cyclo) aliphatic diisocyanates and this polyisocyanate is used as a polyisocyanate component in one- or two-component polyurethane systems, which comprises carrying out the conversion of the (cyclo) aliphatic diisocyanate to the isocyanurate-comprising polyisocyanate in the presence of at least one trimerization catalyst selected from the group of ammonium salts, substituted by four hydrocarbon radicals, of α-hydroxycarboxylates according to any one of claims 1 to 10.

## Revendications

1. Procédé pour la préparation de polyisocyanates contenant des groupes isocyanurate par trimérisation au moins partielle de diisocyanates (cyclo) aliphatiques, **caractérisé en ce qu'**on réalise la transformation en présence d'au moins un catalyseur de trimérisation, choisi dans le groupe des sels d'ammonium d'α-hydroxycarboxylates substitués par quatre radicaux hydrocarbonés.

2. Procédé pour la préparation de polyisocyanates contenant des groupes isocyanurate par trimérisation au moins partielle de diisocyanates (cyclo) aliphatiques, **caractérisé en ce qu'**on utilise comme catalyseur de trimérisation au moins un composé de formule (I), 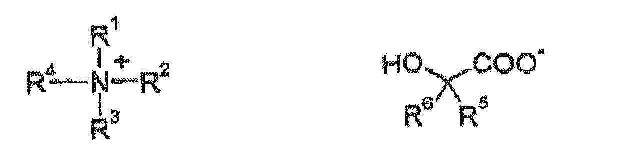 où
R¹, R², R⁴, R⁵ et R⁶ peuvent être, indépendamment l'un de l'autre, à chaque fois identiques ou différents et représentent un groupe alkyle en C₁ à C₂₀ linéaire ou ramifié, un groupe cycloalkyle en C₅ à C₁₂ le cas échéant substitué, un groupe aralkyle en C₇ à C₁₀ le cas échéant substitué ou un groupe aryle en C₆-C₁₂ le cas échéant substitué ou
deux des radicaux R¹ à R⁴ ou plus forment ensemble, l'un avec l'autre, une chaîne alkyle de 4, 5 ou 6 chaînons ou, ensemble avec un atome d'azote, un cycle de 5 ou 6 chaînons, qui peut encore contenir un atome d'azote ou d'oxygène supplémentaire comme élément formant un pont ou ensemble un système polycyclique, de préférence un système bicyclique, à plusieurs chaînons, de préférence à six chaînons, qui peut encore contenir un ou plusieurs atomes d'azote, atomes d'oxygène supplémentaires, ou des atomes d'azote et d'oxygène comme éléments formant un pont et
R⁵ et R⁶ peuvent en outre représenter hydrogène, alkyle en C₁-C₂₀ ou aryle en C₆ à C₁₂, le cas échéant interrompu par un ou plusieurs atomes d'oxygène et/ou de soufre et/ou par un ou plusieurs groupes imino substitués ou non substitués ou le cas échéant substitué par des groupes fonctionnels, aryle, alkyle, aryloxy, alkyloxy, halogène, des hétéroatomes et/ou des hétérocycles.

3. Procédé selon la revendication 2, **caractérisé en ce que** les radicaux R¹ à R⁴ sont choisis, indépendamment l'un de l'autre, dans le groupe constitué par méthyle, éthyle, propyle, isopropyle, n-butyle, tert-butyle, phényle et benzyle.

4. Procédé selon la revendication 2 ou 3, **caractérisé en ce que** les radicaux R⁵ et R⁶ sont choisis, indépendamment l'un de l'autre, dans le groupe constitué par hydrogène, méthyle, éthyle, n-propyle, n-butyle, phényle, 2-carboxyéthyle et 2-hydroxyéthyle.

5. Procédé selon la revendication 1 ou 2, **caractérisé en ce que** l'ion d'ammonium est choisi dans le groupe constitué par tétraoctylammonium, tétraméthylammonium, tétraéthylammonium, tétra-n-butylammonium, triméthylbenzylammonium, triéthylbenzylammonium, tri-n-butylbenzylammonium, triméthyléthylammonium, tri-n-butyléthylammonium, triéthylméthylammonium, tri-n-butylméthylammonium, diiso-propyldiéthylammonium, diiso-propyléthylméthylammonium, diiso-propyléthylbenzylammonium, N,N-diméthylpipéridinium, N,N-diméthylmorpholinium, N,N-diméthylpipérazinium ou N-méthyldiazabicyclo[2,2,2]octane.

6. Procédé selon la revendication 1 ou 2, **caractérisé en ce** qui l'ion d'α-hydroxycarboxylate est choisi dans le groupe constitué par les anions de l'acide glycolique (hydroxyacétique), lactique, citrique, 2-méthyllactique (α-hydroxyisobutyrique), 2-hydroxy-2-méthylbutyrique, 2-hydroxy-2-éthylbutyrique, 2-hydroxy-3-méthylbutyrique, 2-hydroxycaproïque, malique, tartrique, glucuronique, gluconique, citramalique, saccharinique, ribonique, benzylique, quinique, mandélique, hexahydromandélique, 2-hydroxycaproïque et 3-phényllactique.

7. Procédé selon l'une quelconque des revendications précédentes, **caractérisé en ce qu'**on désactive le catalyseur de trimérisation après avoir atteint le degré de trimérisation visé.

8. Procédé selon la revendication 7, **caractérisé en ce qu'**on désactive le catalyseur de trimérisation avec du phosphate de dibutyle ou du phosphate de di-(2-éthylhexyle).

9. Procédé selon l'une quelconque des revendications précédentes, **caractérisé en ce qu'**on utilise, comme diisocyanates, ceux qui présentent une teneur totale en chlore inférieure à 100 ppm en poids.

10. Procédé selon l'une quelconque des revendications précédentes, **caractérisé en ce qu'**on utilise comme diisocyanates le 1,6-hexaméthylènediisocyanate et/ou le 1-isocyanato-3-isocyanatométhyl-3,5,5-triméthylcyclohexane.

11. Utilisation de sels d'ammonium d'α-hydroxycarboxylates substitués par quatre radicaux hydrocarbonés comme catalyseur de trimérisation pour des isocyanates.

12. Procédé pour la préparation de revêtements de polyuréthane, de dispersions de polyuréthane, d'adhésifs à base de polyuréthane et de laques à base de polyuréthane, dans lequel on prépare un polyisocyanate contenant des groupes isocyanurate par trimérisation au moins partielle de diisocyanates (cyclo) aliphatiques et on utilise ce polyisocyanate comme composant de type polyisocyanate dans des systèmes à base de polyuréthane à un ou deux composants, **caractérisé en ce qu'**on réalise la transformation de diisocyanate (cyclo) aliphatique en polyisocyanate contenant des groupes isocyanurate en présence d'au moins un catalyseur de trimérisation, choisi dans le groupe des sels d'ammonium d'α-hydroxycarboxylates substitués par quatre radicaux hydrocarbonés selon l'une quelconque des revendications 1 à 10.
